# EUROPEAN PATENT SPECIFICATION

(11) **EP 1 857 041 B1**
(45) Date of publication and mention of the grant of the patent: **23.09.2009**
(21) Application number: 07252011.7
(22) Date of filing: 16.05.2007
(51) Int. Cl.: A61B 1/018, A61M 25/01, A61B 1/005, A61B 1/31

(54) **Medical instrument having a catheter and having a catheter accessory device**
Medizinisches Instrument mit einem Katheter und einem Zusatzgerät für den Katheter
Instrument médical comportant un cathéter et un dispositif d'accessoire de cathéter

(30) Priority: 17.05.2006 US 435551
(43) Date of publication of application: 21.11.2007
(73) Proprietor: ETHICON ENDO-SURGERY, INC., Cincinnati, Ohio 45242 (US)
(72) Inventor: Long, Gary L., Cincinnati Ohio 45227 (US)
(74) Representative: Tunstall, Christopher Stephen

(56) References cited:
- WO-A-01/93938
- WO-A-02/43797
- WO-A-03/053225
- WO-A-20/05113051
- US-A- 5 353 807
- US-A- 5 706 827
- US-A- 5 776 080

## Description

**Field of the Invention**

The present invention is related generally to medical equipment, and more particularly to a medical instrument having a catheter and having a catheter accessory device.

**Background of the Invention**

Examples of known catheters include, without limitation, flexible insertion tubes of endoscopes (including flexible insertion tubes of colonoscopes and enteroscopes). The insertion tube has an articulatable distal end portion controlled by wires running from the distal end portion to control knobs on the handle of the endoscope. A wide angle video camera in the distal end of the insertion tube permits medical observation. In use, the distal end of the insertion tube is inserted into a body lumen of a patient. The user manually pushes on a proximal tube portion to advance the distal end of the insertion tube within the body lumen for medical observation and/or medical treatment. In a serpentine body lumen, such as the colon, the articulatable distal end of the insertion tube can become misaligned in the body lumen and become blocked by lumen tissue from further advancement. Then, if the user further pushes on the proximal tube portion, the insertion tube forms undesirable loops which the user must correct before realigning the distal end of the insertion tube and further advancing the insertion tube within the body lumen.

Typically, an accessory medical device is part of an endoscopic system. In one example, the accessory device is an end effector (such as a medical snare, a medical grasper, etc.) which is inserted into a working channel of the insertion tube of the endoscope and is manually translated to extend from the distal end of the working channel for medical observation and/or medical treatment. In another example, the accessory device is a flexible accessory tube adapted to be coupled to, and manually translated along, an exterior rail of the insertion tube of an endoscope after the insertion tube has been advanced to a target site within the body lumen. Then, an end effector is inserted into an accessory working channel of the accessory tube and is manually translated to extend from the distal end of the accessory working channel for medical observation and/or medical treatment. Pushing on the proximal end of the accessory tube to translate the accessory tube along the exterior rail can also lead to the formation of undesirable loops.

It is known to manually advance a guidewire within a body lumen and then to manually advance a catheter along the guidewire.

US5706827 discusses a catheter configured for use with a guidewire that includes operably connected, magnetically responsive material. The catheter comprises an elongated shaft having a proximal end and a distal end. Magnetically active material is operably connected to the shaft and configured such that the shaft may be magnetically coupled to the guidewire, along a portion of the shaft.

W02005113051 discusses a first medical instrument which includes a flexible catheter and a medical guidewire extendable beyond a distal end of the catheter. The medical guidewire has first and second segments, wherein the bending moment of inertia of the first segment is less than the bending moment of inertia of the second segment. A second medical instrument includes a flexible catheter, a mechanized guidewire assembly, and a medical guidewire extendable beyond a distal end of the catheter.

W003053225 discusses a device which includes an elongate body with a movable mass arranged for sliding movement within the body, the element being arranged to decelerate toward an end of the body in order to impart forward movement thereto, via momentum transfer, and being arranged to accelerate away from said end in order to further drive the body forward, again using momentum transfer.

Still, scientists and engineers continue to seek improved medical instruments having a catheter and having a catheter accessory device.

**Summary of the Invention**

A first expression of an embodiment of the invention is for a medical instrument including a flexible medical catheter and a catheter accessory device. The catheter has a distal end insertable within a body lumen of a patient. The device is slidably engagable with the catheter. One of the catheter and the device includes magnetic material, and the other of the catheter and the device includes electrically-energizable windings. The catheter and the device are adapted to operate as a linear motor wherein controllably electrically energizing the windings while immobilizing one of the catheter and the device slidingly moves the other of the catheter and the device.

A second expression of an embodiment of the invention is for a medical instrument including a flexible medical catheter, a catheter accessory device, and a linear motor controller. The catheter has a distal end insertable within a body lumen of a patient. The device slidably engages the catheter. One of the catheter and the device includes magnetic material, and the other of the catheter and the device includes electrically-energizable windings. The catheter and the device are adapted to operate as a linear motor wherein controllably electrically energizing the windings while immobilizing one of the catheter and the device slidingly moves the other of the catheter and the device. The controller is operatively connected to the windings.

As an example, a method is for using a medical instrument. The medical instrument includes a flexible medical catheter, a catheter accessory device, and a linear motor controller. The catheter has a distal end insertable within a body lumen of a patient. The device slidably engages the catheter. One of the catheter and the device includes magnetic material, and the other of the catheter and the device includes electrically-energizable windings. The catheter and the device are adapted to operate as a linear motor wherein controllably electrically energizing the windings while immobilizing one of the catheter and the device slidingly moves the other of the catheter and the device. The controller is operatively connected to the windings. The method includes inserting the distal end within the body lumen. The method also includes immobilizing one of the catheter and the device. The method also includes activating the controller to advance the other of the catheter and the device within the body lumen.

Several benefits and advantages are obtained from one or more of the expressions of an embodiment of the invention. In a first example, the device is a medical guidewire which has been immobilized after having been manually advanced within the body lumen to extend from the catheter wherein controllably electrically energizing the windings slidingly moves the catheter along the guidewire. In one illustration of this example, the force to advance the catheter is applied proximate the distal end of the catheter so that the self-advancing catheter will not form undesirable loops, as can be appreciated by those skilled in the art. In a second example, the device includes a flexible accessory tube, and the catheter is immobilized after having been manually advanced within the body lumen wherein controllably electrically energizing the windings slidingly moves the accessory tube along an exterior rail of the catheter. In one illustration of this example, the force to advance the accessory tube is applied proximate the distal end of the accessory tube so that the self-advancing accessory tube will not form undesirable loops, as can be appreciated by those skilled in the art. In one employment of either or both examples, the self-advancing catheter and/or the self-advancing accessory tube has a greater advancement speed, than that obtained with manual advancement, which reduces the time required for a medical procedure.

**Brief Description of the Figures**

FIGURE 1 is a schematic view of an embodiment of a medical instrument including a medical catheter, a catheter accessory device in the form of a catheter-guiding member which is a catheter guide wire, and a controller, wherein the catheter includes windings, wherein the guidewire includes magnetic material, and wherein the controller is located in a handpiece which is shown in cut-away;

FIGURE 2 is a cross sectional view of the distal portion of the medical catheter of Figure 1 inserted within a body lumen of a patient, wherein the medical catheter of Figure 2 has been rotated ninety degrees from the orientation shown in Figure 1;

FIGURE 3 is a schematic wiring diagram showing the controller electrically connected to the windings;

FIGURE 4 is a perspective view of a distal portion of a first alternate embodiment of the medical instrument of Figure 1, wherein the catheter-guiding member is a catheter guide ribbon;

FIGURE 5 is side elevational view of a distal portion of a second alternate embodiment of the medical instrument of Figure 1, wherein the catheter includes an exterior rail and wherein the catheter accessory device is adapted to slide along the exterior rail and includes a flexible accessory tube having an accessory working channel;

FIGURE 6 is a view taken along lines 6-6 of Figure 5; and

FIGURE 7 is a cross sectional view of the catheter accessory device of Figure 6 taken along lines 7-7 of Figure 6.

**Detailed Description of the Invention**

Before explaining the present invention in detail, it should be noted that the invention is not limited in its application or use to the details of construction and arrangement of parts illustrated in the accompanying drawings and description. The illustrative embodiments of the invention may be implemented or incorporated in other embodiments, variations and modifications, and may be practiced or carried out in various ways. Furthermore, unless otherwise indicated, the terms and expressions employed herein have been chosen for the purpose of describing the illustrative embodiments of the present invention for the convenience of the reader and are not for the purpose of limiting the invention.

It is understood that any one or more of the following-described expressions, embodiments, examples, etc. can be combined with any one or more of the other following-described expressions, embodiments, examples, etc.

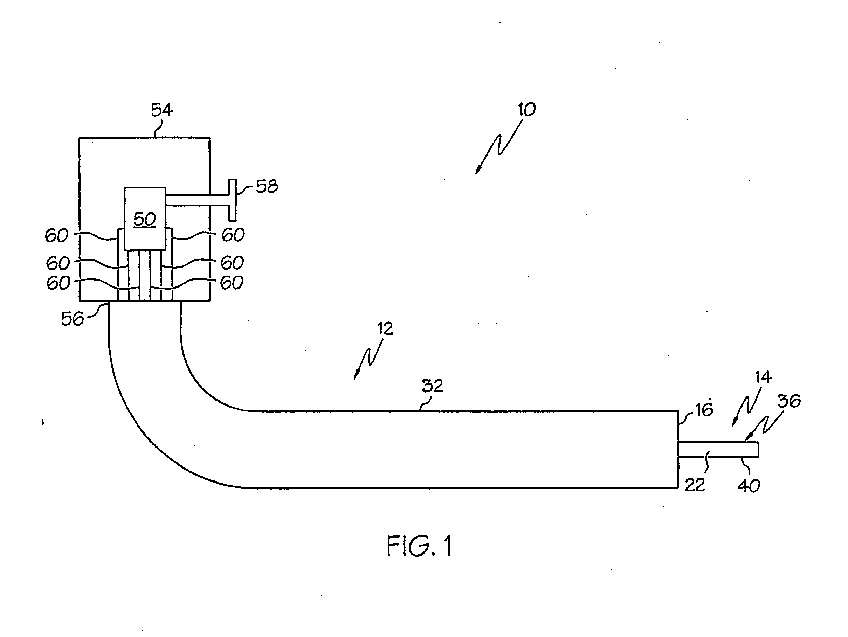

Referring now to the Figures, wherein like numerals represent like elements throughout, Figures 1-3 illustrate an embodiment of the invention. A first expression of the embodiment of Figures 1-3 is for a medical instrument 10 including a flexible medical catheter 12 and a catheter accessory device 14. The catheter 12 has a distal end 16 insertable within a body lumen 18 of a patient 20. The device 14 is slidably engagable with the catheter 12. One of the catheter 12 and the device 14 includes magnetic material 22, and the other of the catheter 12 and the device 14 includes electrically-energizable windings 24, 26 and 28. The catheter 12 and the device 14 are adapted to operate as a linear motor 30 wherein controllably electrically energizing the windings 24, 26 and 28 while immobilizing one of the catheter 12 and the device 14 slidingly moves the other of the catheter 12 and the device 14.

It is noted that the terminology "magnetic material" includes material capable of being magnetized and includes permanent magnets. It is also noted that various designs and the operation of a linear motor are well known in the art. The number of turns in a winding and the number and arrangement of the windings are left to the artisan.

In one application of the first expression of the embodiment of Figures 1-3, the catheter 12 is a flexible endoscope insertion tube 32. In one variation, the flexible endoscope insertion tube 32 has a single working channel 34. Non-endoscope applications are left to those skilled in the art.

In a first implementation of the first expression of the embodiment of Figures 1-3, the device 14 is a flexible catheter-guiding member 36. In one variation, the catheter 12 includes a catheter lumen 38, and the member 36 is disposable in the catheter lumen 38. In one modification, the catheter 12 includes the windings 24, 26 and 28, and the windings 24, 26 and 28 are disposed proximate the distal end 16 of the catheter 12. In a first example, the member 36 is a catheter guide wire 40. In one choice of materials, the guide wire 40 comprises iron.

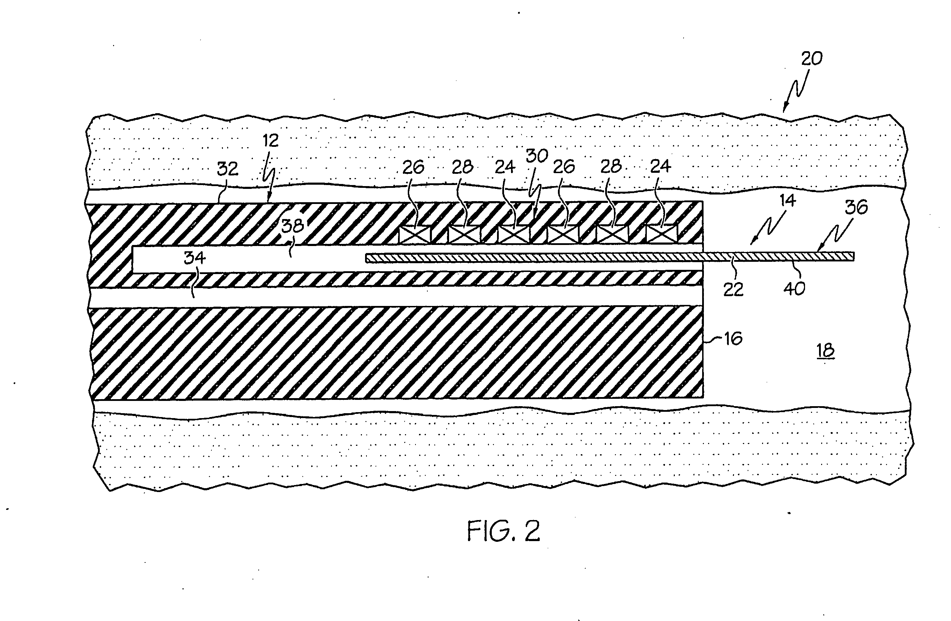

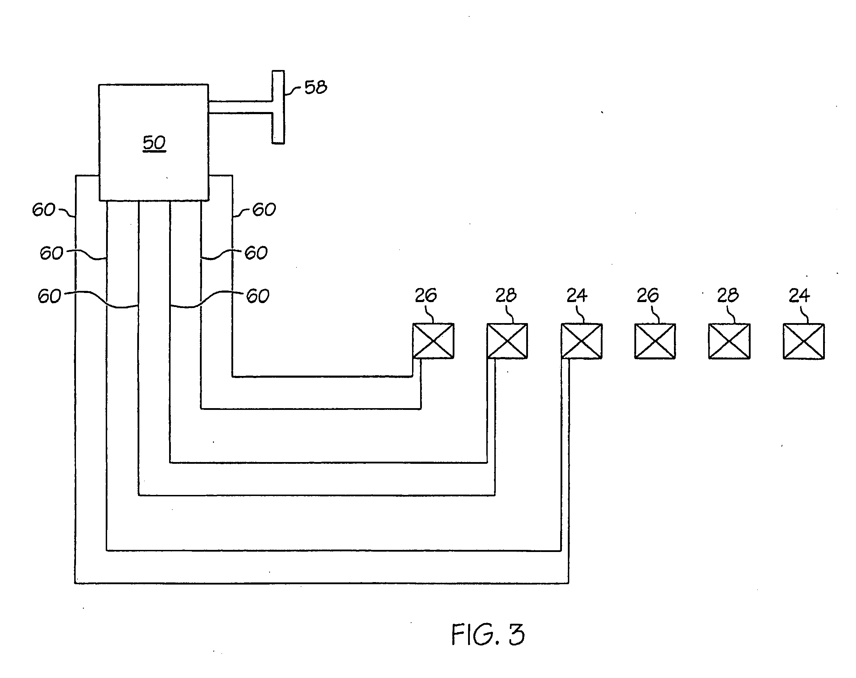

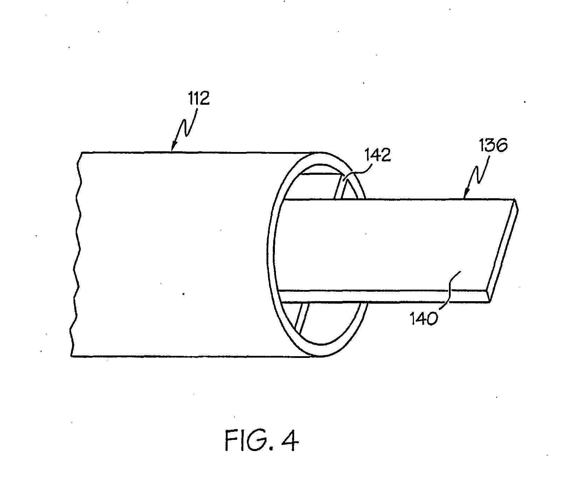

In a second example, shown in the first alternate embodiment of Figure 4, the member 136 is a catheter guide ribbon 140 which provides a larger area allowing for a larger magnetomotive force as can be appreciated by the artisan. In one variation, the guide ribbon 140 slidingly engages a lumen-dividing wall 142 within the catheter 112. In one modification, the wall 142 includes the windings (not shown in Figure 3, but a cross sectional view of Figure 3 showing the windings would look similar to the view of Figure 2). In one choice of materials, the guide ribbon 140 comprises iron.

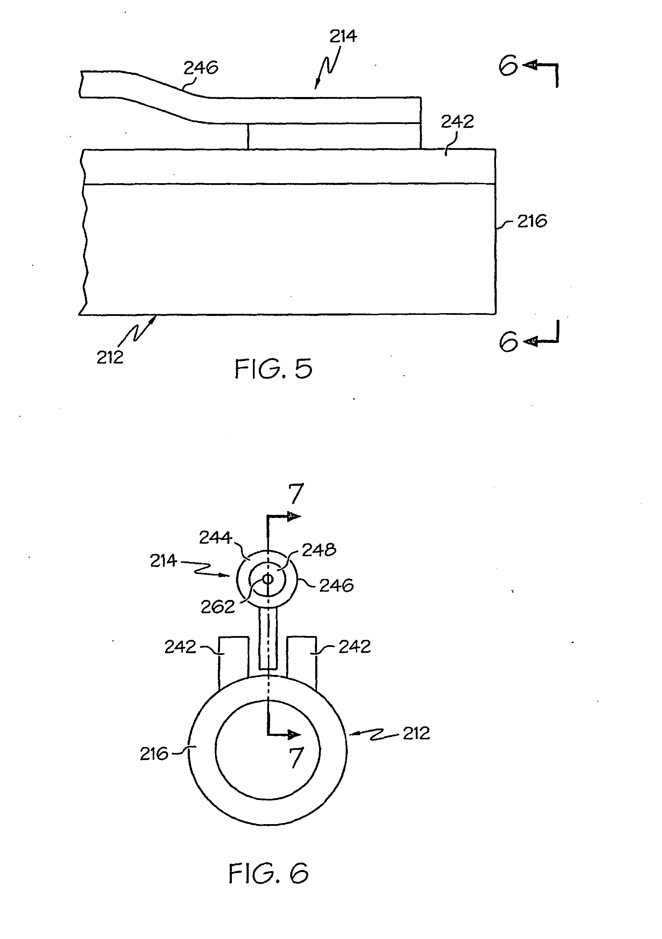

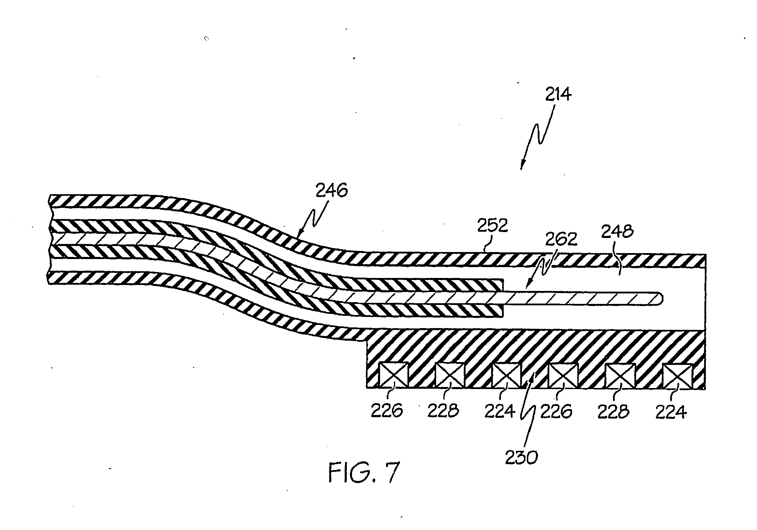

In a second implementation, as shown in the second alternate embodiment of Figures 5-7, the catheter 212 includes an exterior rail 242, and the device 214 is adapted to slide along the exterior rail 242 during operation of the linear motor 230. In one variation, the device 214 has a distal end 244, the device 214 includes the windings 224, 226 and 228, and the windings 224, 226 and 228 are disposed proximate the distal end 244 of the device 214. In one example, the device 214 includes a flexible accessory tube 246 having an accessory working channel 248. In one choice of materials, the exterior rail 242 comprises an elastomer having iron particles (omitted from Figure 7 for clarity) embedded therein. In a first construction, the device 214 is coupled to the exterior rail 242 by magnetic force, and a stop (not shown) at the distal end 216 of the catheter 212 limits travel of the accessory tube 246. In a second construction, not shown, the device is coupled to the exterior rail by a tongue and groove arrangement. Other coupling constructions are left to the artisan.

A second expression of the embodiment of Figures 1-3 is for a medical instrument 10 including a flexible medical catheter 12, a catheter accessory device 14, and a linear motor controller 50. The catheter 12 has a distal end 16 insertable within a body lumen 18 of a patient 20. The device 14 slidably engages the catheter 12. One of the catheter 12 and the device 14 includes magnetic material 22, and the other of the catheter 12 and the device 14 includes electrically-energizable windings 24, 26 and 28. The catheter 12 and the device 14 are adapted to operate as a linear motor 30 wherein controllably electrically energizing the windings 24, 26 and 28 while immobilizing one of the catheter 12 and the device 14 slidingly moves the other of the catheter 12 and the device 14. The controller 50 is operatively connected to the windings 24, 26 and 28.

In one application of the second expression of the embodiment of Figures 1-3, the catheter 12 is a flexible endoscope insertion tube 32.

In a first implementation of the second expression of the embodiment of Figures 1-3, the device 14 is a flexible catheter-guiding member 36. In one variation, the catheter 12 includes a catheter lumen 38, and the member 36 is disposed in the catheter lumen 38. In one employment, the controller 50, when activated by a user, controllably electrically energizes the windings 24, 26 and 28 to slidingly move the catheter 12 along the member 36 when the member 36 is immobilized by the user.

In a second implementation, as shown in the second alternate embodiment of Figures 5-7, the catheter 212 includes an exterior rail 242, and the device 214 is adapted to slide along the exterior rail 242 during activation of the controller (the controller is shown as controller 50 in Figures 1 and 3). In one example, the device 214 includes a flexible accessory tube 246 having an accessory working channel 248 and a distal accessory tube portion 252, wherein the controller (the controller is shown as controller 50 in Figures 1 and 3), when activated by a user, controllably electrically energizes the windings 224, 226 and 228 to slidingly move the distal accessory tube portion 252 along the exterior rail 242 when the catheter 212 is immobilized by the user.

In one enablement of the first and/or second expressions of the embodiment of Figures 1-3, the medical instrument 10 includes a handpiece 54 attached to the proximal end 56 of the catheter 12, wherein the handpiece 54 includes the controller 50 with the controller 50 having a button 58 for a user to activate the controller 50. In one variation, as shown in Figure 3, wires 60 connect the controller 50 to the windings 24, 26 and 28. In one modification of the example of the flexible accessory tube 246 having the accessory working channel 248, an end effector 262, such as a medical needle knife, is manually translatable within the accessory working channel 248 to extend from the accessory tube 246 for medical observation and/or medical treatment of the patient. Other types of medical end effectors include, without limitation, imagers, irrigators, cutting blades, wire snares, and ultrasound transducers.

An examplary method is for using a medical instrument 10. The medical instrument 10 includes a flexible medical catheter 12, a catheter accessory device 14, and a linear motor controller 50. The catheter 12 has a distal end 16 insertable within a body lumen 18 of a patient 20. The device 14 slidably engages the catheter 12. One of the catheter 12 and the device 14 includes magnetic material 22, and the other of the catheter 12 and the device 14 includes electrically-energizable windings 24, 26 and 28. The catheter 12 and the device 14 are adapted to operate as a linear motor 30 wherein controllably electrically energizing the windings 24, 26 and 28 while immobilizing one of the catheter 12 and the device 14 slidingly moves the other of the catheter 12 and the device 14. The controller 50 is operatively connected to the windings 24, 26 and 28. The method includes inserting the distal end 16 of the catheter 12 within the body lumen 18. The method also includes immobilizing one of the catheter 12 and the device 14. The method also includes activating the controller 50 to advance the other of the catheter 12 and the device 14 within the body lumen 18.

In a first employment of the examplary method, the device 14 is a flexible catheter-guiding member 36, wherein the catheter 12 includes a catheter lumen 38, wherein the member 36 is disposed in the catheter lumen 38, and wherein activating the controller 50 controllably electrically energizes the windings 24, 26 and 28 to slidingly move the catheter 12 along the member 36 when the member 36 is immobilized. In one extension, the method also includes manually advancing the member 36 slidingly through the catheter lumen 38 before immobilizing the member 36 and activating the controller 50.

In a second employment of the examplary method, the catheter 212 includes an exterior rail 242, wherein activating the controller (the controller is shown as controller 50 in Figures 1 and 3) slidingly moves the device 214 along the exterior rail 242 when the catheter 212 is immobilized. In one extension, the method also includes manually advancing the catheter 212 before immobilizing the catheter 212 and activating the controller.

In one utilization of the examplary method, the body lumen is a colon of a human or other mammal. In another utilization, the body lumen is an upper gastrointestinal tract. In a further utilization, the body lumen is an artery lumen. Other body lumens are left to those skilled in the art.

Several benefits and advantages are obtained from one or more of the expressions of an embodiment of the invention. In a first example, the device is a medical guidewire which has been immobilized after having been manually advanced within the body lumen to extend from the catheter wherein controllably electrically energizing the windings slidingly moves the catheter along the guidewire. In one illustration of this example, the force to advance the catheter is applied proximate the distal end of the catheter so that the self-advancing catheter will not form undesirable loops, as can be appreciated by those skilled in the art. In a second example, the device includes a flexible accessory tube, and the catheter is immobilized after having been manually advanced within the body lumen wherein controllably electrically energizing the windings slidingly moves the accessory tube along an exterior rail of the catheter. In one illustration of this example, the force to advance the accessory tube is applied proximate the distal end of the accessory tube so that the self-advancing accessory tube will not form undesirable loops, as can be appreciated by those skilled in the art. In one exemplary employment of either or both examples, the self-advancing catheter and/or the self-advancing accessory tube has a greater advancement speed, than that obtained with manual advancement, which reduces the time required for a medical procedure.

While the present invention has been illustrated by a description of several expressions of embodiments, it is not the intention of the applicants to restrict or limit the scope of the appended claims to such detail. Numerous other variations, changes, and substitutions will occur to those skilled in the art without departing from the scope of the invention. For instance, the medical instrument of the invention has application in robotic assisted surgery taking into account the obvious modifications of such systems and devices to be compatible with such a robotic system. It will be understood that the foregoing description is provided by way of example, and that other modifications may occur to those skilled in the art without departing from the scope of the appended Claims.

## Claims

1. A medical instrument (10) comprising:
a) a flexible medical catheter (12) having a distal end (16) insertable within a body lumen (18) of a patient (20); and
b) a catheter accessory device (14) slidably engagable with the catheter, wherein one of the catheter and the device includes magnetic material (22), wherein the other of the catheter and the device includes electrically-energizable windings (24, 26, 28), and
**characterized in that**:
the catheter and the device are adapted to operate as a linear motor (30) wherein controllably electrically energizing the windings while immobilizing one of the catheter and the device slidingly moves the other of the catheter and the device.

2. The medical instrument of claim 1, wherein the catheter is a flexible endoscope insertion tube (32).

3. The medical instrument of claim 1, wherein the device is a flexible catheter-guiding member (36).

4. The medical instrument of claim 3, wherein the catheter includes a catheter lumen (38), and wherein the member is disposable in the catheter lumen.

5. The medical instrument of claim 4, wherein the catheter includes the windings, and wherein the windings are disposed proximate the distal end of the catheter.

6. The medical instrument of claim 5, wherein the member is chosen from the group consisting of a catheter guide wire (40) and a catheter guide ribbon (140).

7. The medical instrument of claim 1, wherein the catheter (212) includes an exterior rail (242), and wherein the device is adapted to slide along the exterior rail during operation of the linear motor (230).

8. The medical instrument of claim 7, wherein the device has a distal end, wherein the device includes the windings, and wherein the windings are disposed proximate the distal end of the device.

9. The medical instrument of claim 8, wherein the device includes a flexible accessory tube (246) having an accessory working channel (248).

10. The medical instrument of claim 1, further comprising:
c) a linear motor controller (50) operatively connected to the windings.

11. The medical instrument of claim 10, wherein the catheter includes a catheter lumen, wherein the member is disposed in the catheter lumen, and wherein the controller, when activated by a user, controllably electrically energizes the windings to slidingly move the catheter along the member when the member is immobilized by the user.

12. The medical instrument of claim 9, wherein the catheter includes an exterior rail, and wherein the device is adapted to slide along the exterior rail during activation of the controller.

13. The medical instrument of claim 12, wherein the device includes a flexible accessory tube having an accessory working channel and a distal accessory tube portion, and wherein the controller, when activated by a user, controllably electrically energizes the windings to slidingly move the distal accessory tube portion along the exterior rail when the catheter is immobilized by the user.

## Patentansprüche

1. Medizinisches Instrument (10), umfassend:
a) einen flexiblen medizinischen Katheter (12) mit einem distalen Ende (16), das in ein Körperlumen (18) eines Patienten (20) einsetzbar ist; und
b) eine Zusatzeinrichtung (14) für einen Katheter, die mit dem Katheter gleitfähig in Eingriff bringbar ist, wobei eine(r) von dem Katheter und der Einrichtung magnetisches Material (22) enthält und der andere/die andere von dem Katheter und der Einrichtung mit elektrischem Strom versorgbare Wicklungen (24, 26, 28) enthält,
**dadurch gekennzeichnet, dass**
der Katheter und die Einrichtung gestaltet sind, um als ein Linearmotor (30) zu arbeiten, wobei steuerbares Versorgen der Wicklungen mit elektrischem Strom bei Immobilisierung von einem/einer von dem Katheter und der Einrichtung den anderen/die andere von dem Katheter und der Einrichtung gleitend bewegt.

2. Medizinisches Instrument nach Anspruch 1, **dadurch gekennzeichnet, dass** der Katheter ein flexibles Endoskopeinführrohr (32) ist.

3. Medizinisches Instrument nach Anspruch 1, **dadurch gekennzeichnet, dass** die Einrichtung ein flexibles Katheterführungselement (36) ist.

4. Medizinisches Instrument nach Anspruch 3, **dadurch gekennzeichnet, dass** der Katheter ein Katheterlumen (38) enthält und das Element in dem Katheterlumen anordbar ist.

5. Medizinisches Instrument nach Anspruch 4, **dadurch gekennzeichnet**, der Katheter die Wicklungen enthält und die Wicklungen in der Nähe des distalen Ende des Katheters angeordnet sind.

6. Medizinisches Instrument nach Anspruch 5, **dadurch gekennzeichnet, dass** das Element aus der Gruppe ausgewählt ist, die aus einem Katheterführungsdraht (40) und einem Katheterführungsband (140) besteht.

7. Medizinisches Instrument nach Anspruch 1, **dadurch gekennzeichnet, dass** der Katheter (212) eine äußere Schiene (242) enthält und die Einrichtung gestaltet ist, um entlang der äußeren Schiene während des Betriebs des Linearmotors (230) zu gleiten.

8. Medizinisches Instrument nach Anspruch 7, **dadurch gekennzeichnet, dass** die Einrichtung ein distales Ende aufweist, die Einrichtung die Wicklungen enthält und die Wicklungen in der Nähe des distalen Endes der Einrichtung angeordnet sind.

9. Medizinisches Instrument nach Anspruch 8, **dadurch gekennzeichnet, dass** die Einrichtung ein flexibles Zusatzrohr (246) mit einen zusätzlichen Arbeitskanal (245) enthält.

10. Medizinisches Instrument nach Anspruch 1, ferner umfassend:
c) eine Linearmotorsteuerung (50), die mit den Wicklungen in Wirkverbindung steht.

11. Medizinisches Instrument nach Anspruch 10, **dadurch gekennzeichnet, dass** der Katheter ein Katheterlumen enthält, das Element in dem Katheterlumen angeordnet ist und die Steuerung bei Aktivierung durch einen Benutzer die Wicklungen steuerbar mit elektrischem Strom versorgt, um den Katheter entlang des Elements gleitend zu bewegen, wenn das Element durch den Benutzer immobilisiert ist.

12. Medizinisches Instrument nach Anspruch 9, **dadurch gekennzeichnet, dass** der Katheter eine äußere Schiene enthält und die Einrichtung gestaltet ist, um entlang der äußeren Schiene während Aktivierung der Steuerung zu gleiten.

13. Medizinisches Instrument nach Anspruch 12, **dadurch gekennzeichnet** die Einrichtung ein flexibles Zusatzrohr mit einem zusätzlichen Arbeitskanal und einem distalen Zusatzrohrabschnitt enthält und die Steuerung bei Aktivierung durch einen Benutzer die Wicklungen steuerbar mit elektrischem Strom versorgt, um den distalen Zusatzrohrabschnitt entlang der äußeren Schiene gleitend zu bewegen, wenn der Katheter durch den Benutzer immobilisiert ist.

## Revendications

1. Instrument médical (10) comprenant :
a) un cathéter médical flexible (12) ayant une extrémité distale (16) insérable à l'intérieur d'une lumière corporelle (18) d'un patient (20) ; et
b) un dispositif d'accessoire de cathéter (14) pouvant se mettre en prise de façon coulissante avec le cathéter, un du cathéter et du dispositif comprenant un matériau magnétique (22), l'autre du cathéter et du dispositif comprenant des enroulements excitables électriquement (24, 26, 28), et
**caractérisé en ce que** :
le cathéter et le dispositif sont conçus pour fonctionner comme un moteur linéaire (30), l'excitation électrique contrôlable des enroulements tout en immobilisant un du cathéter et du dispositif permettant le déplacement coulissant de l'autre du cathéter et du dispositif.

2. Instrument médical selon la revendication 1, dans lequel le cathéter est un tube d'insertion d'endoscope flexible (32).

3. Instrument médical selon la revendication 1, dans lequel le dispositif est un élément de guidage de cathéter flexible (36).

4. Instrument médical selon la revendication 3, dans lequel le cathéter comprend une lumière de cathéter (38), et dans lequel l'élément peut être disposé dans la lumière de cathéter.

5. Instrument médical selon la revendication 4, dans lequel le cathéter comprend les enroulements, et dans lequel les enroulements sont disposés à proximité de l'extrémité distale du cathéter.

6. Instrument médical selon la revendication 5, dans lequel l'élément est choisi dans le groupe comprenant un fil-guide de cathéter (40) et un ruban-guide de cathéter (140).

7. Instrument médical selon la revendication 1, dans lequel le cathéter (212) comprend un rail extérieur (242), et dans lequel le dispositif est conçu pour coulisser le long du rail extérieur pendant le fonctionnement du moteur linéaire (230).

8. Instrument médical selon la revendication 7, dans lequel le dispositif a une extrémité distale, dans lequel le dispositif comprend les enroulements, et dans lequel les enroulements sont disposés à proximité de l'extrémité distale du dispositif.

9. Instrument médical selon la revendication 8, dans lequel le dispositif comprend un tube d'accessoire flexible (246) ayant un canal opérateur d'accessoire (248).

10. Instrument médical selon la revendication 1, comprenant en outré :
c) un contrôleur de moteur linéaire (50) connecté de façon opérationnelle aux enroulements.

11. Instrument médical selon la revendication 10, dans lequel le cathéter comprend une lumière de cathéter, dans lequel l'élément est disposé dans la lumière de cathéter, et dans lequel le contrôleur, lorsqu'il est activé par un utilisateur, excite électriquement de façon contrôlable les enroulements pour déplacer de façon coulissante le cathéter le long de l'élément lorsque l'élément est immobilisé par l'utilisateur.

12. Instrument médical selon la revendication 9, dans lequel le cathéter comprend un rail extérieur, et dans lequel le dispositif est conçu pour coulisser le long du rail extérieur pendant l'activation du contrôleur.

13. Instrument médical selon la revendication 12, dans lequel le dispositif comprend un tube d'accessoire flexible ayant un canal opérateur d'accessoire et une partie de tube accessoire distale, et dans lequel le contrôleur, lorsqu'il est activé par un utilisateur, excite électriquement de façon contrôlable les enroulements pour déplacer de façon coulissante la partie de tube d'accessoire distale le long du rail extérieur lorsque le cathéter est immobilisé par l'utilisateur.
